(19) Europäisches Patentamt
European Patent Office
Office européen des brevets

(11) **EP 4 406 479 A1**

(12) **EUROPEAN PATENT APPLICATION**
published in accordance with Art. 153(4) EPC

(43) Date of publication:
**31.07.2024 Bulletin 2024/31**

(21) Application number: **22872819.2**

(22) Date of filing: **15.09.2022**

(51) International Patent Classification (IPC):
***A61B 5/291*** *(2021.01)* ***A61B 5/256*** *(2021.01)*

(52) Cooperative Patent Classification (CPC):
**A61B 5/256; A61B 5/291**

(86) International application number:
**PCT/JP2022/034608**

(87) International publication number:
**WO 2023/048065 (30.03.2023 Gazette 2023/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.09.2021 JP 2021155434**

(71) Applicant: **Sumitomo Bakelite Co.Ltd.**
**Shinagawa-ku**
**Tokyo 140-0002 (JP)**

(72) Inventor: **SAWADA, Masashi**
**Tokyo 140-0002 (JP)**

(74) Representative: **Ter Meer Steinmeister & Partner**
**Patentanwälte mbB**
**Nymphenburger Straße 4**
**80335 München (DE)**

(54) **BRAIN WAVE DETECTION ELECTRODE, BRAIN WAVE MEASUREMENT DEVICE, AND BRAIN WAVE MEASUREMENT METHOD**

(57) Provided is a brain wave detection electrode (50) which is brought into contact with a scalp of a subject to detect brain waves, the brain wave detection electrode including a base portion (51), a plurality of pyramid-shaped protrusion portions (60) protruding from the base portion (51), and an electrode portion provided in the protrusion portion (60), in which a distance D1 between protrusion portions (60) most adjacent to each other among the plurality of protrusion portions (60) satisfies the following expression (1).

$$D1 < L(m^2 + n^2)^{1/2} - d \text{ or } D1 > L(m^2 + n^2)^{1/2} + d \quad \ldots \quad (1)$$

m and n: an integer of 0 or more, in which either m or n is 1 or more,
L: a distance between centers of hair follicles in the scalp of the subject, and
d: a diameter of the hair follicle in the scalp of the subject

FIG. 6

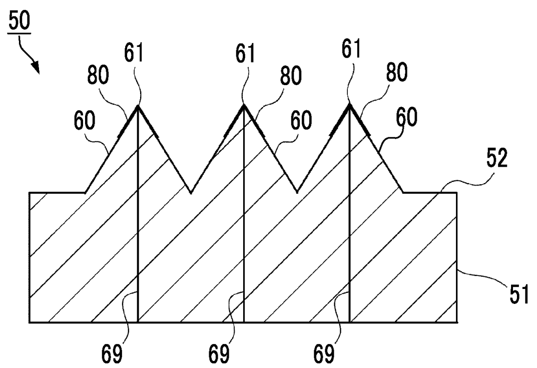


EP 4 406 479 A1

**Description**

TECHNICAL FIELD

The present invention relates to a brain wave detection electrode, a brain wave measuring device, and a brain wave measuring method.

BACKGROUND ART

Various developments have been made so far regarding a brain wave detection electrode. As a technique of this kind, for example, techniques disclosed in Patent Documents 1 to 3 have been known.

Patent Document 1 discloses a brain wave measuring electrode (brain wave detection electrode) including a base portion, a protruding portion made of rubber and provided to be protruded from the base portion, and a contact portion made of metal and provided at a tip of the protruding portion, the contact portion being electrically connected to an outside of the brain wave measuring electrode and coming into contact with the scalp during the measurement of the brain waves.

Patent Document 2 discloses a biological electrode including an electrode member which is in contact with a body of a subject, and a conductive support member which supports the electrode member, in which at least the electrode member is made of a conductive rubber, the conductive rubber contains silicone rubber and silver powder, a plurality of the electrode members are provided so as to protrude from the support member in a brush shape, and the biological electrode is used for measuring brain waves.

Patent Document 3 discloses a brain wave measuring electrode including a base material made of an elastic body and a structural body formed on a surface of the base material, in which the base material includes a protruding portion having a contact surface which comes into contact with the scalp, the structural body contains a plurality of nano-carbon materials, the plurality of nano-carbon materials form a network structure in which the nano-carbon materials are connected to each other, and the structure is fixed to the surface of the base material.

RELATED DOCUMENT

PATENT DOCUMENT

[Patent Document 1] Japanese Patent No. 5842198
[Patent Document 2] International Publication No. 2021/029118
[Patent Document 3] International Publication No. 2016/136629

SUMMARY OF THE INVENTION

TECHNICAL PROBLEM

In a case where the brain wave electrode has a configuration in which a plurality of protrusion portions having an electrode portion at a tip are provided, there is a problem in that, when the vertexes (tips) of all the protrusion portions are placed on the hair follicles of the scalp, the electrode portion comes into contact with the hair, a contact surface with the scalp is lost, and the brain waves cannot be acquired or the acquired brain waves are not stable.

The techniques of Patent Documents 1 to 3 do not consider the above-described problems, and a new technique has been required.

The present invention has been made in view of such circumstances, and an object of the present invention is to provide a technique of a brain wave electrode having a configuration including a plurality of protrusion portions each having an electrode portion at a tip, in which any of the tips of the protrusion portions is not placed on a hair follicle so that the brain wave electrode can be reliably in contact with a scalp.

SOLUTION TO PROBLEM

According to the present invention, the following inventions are provided.

[1] A brain wave detection electrode which is brought into contact with a scalp of a subject to detect brain waves, the brain wave detection electrode including:

a base portion;
a plurality of pyramid-shaped protrusion portions protruding from the base portion; and
an electrode portion provided in the protrusion portion,
in which a distance D1 between protrusion portions most adjacent to each other among the plurality of protrusion portions satisfies the following expression (1),

$$D1 < L(m^2 + n^2)^{1/2} - d \text{ or } D1 > L(m^2 + n^2)^{1/2} + d \quad \ldots \quad (1)$$

m and n: an integer of 0 or more, in which either m or n is 1 or more,
L: a distance between centers of hair follicles in the scalp of the subject, and
d: a diameter of the hair follicle in the scalp of the subject.

[2] The brain wave detection electrode according to [1],
in which the plurality of protrusion portions are arranged with a configuration in which vertices of the plurality of protrusion portions form a square lattice.
[3] The brain wave detection electrode according to [1],
in which the plurality of protrusion portions are arranged with a configuration in which vertices of the plurality of protrusion portions form an equilateral triangle lattice.
[4] The brain wave detection electrode according to any one of [1] to [3],
in which the protrusion portion is a pyramid.
[5] The brain wave detection electrode according to [4],
in which the pyramid of the protrusion portion is a hexagonal pyramid.
[6] The brain wave detection electrode according to [5],
in which the protrusion portions are arranged such that sides of bottom surfaces of adjacent protrusion portions are in contact with each other.
[7] The brain wave detection electrode according to [6],
in which the pyramid of the protrusion portion is a cone.
[8] The brain wave detection electrode according to any one of [1] to [7],
in which the distance D1 between the protrusion portions most adjacent to each other is 2 mm or more and 10 mm or less.
[9] A brain wave measuring device including:

the brain wave detection electrode according to any one of [1] to [8]; and
a frame to which the brain wave detection electrode is attached, the frame being mounted on the head of the subject.

[10] A brain wave measuring method including:
mounting the brain wave measuring device according to [9] on the head of the subject and measuring brain waves.

## ADVANTAGEOUS EFFECTS OF INVENTION

According to the present invention, it is possible to provide a technique of a brain wave electrode having a configuration including a plurality of protrusion portions each having an electrode portion at a tip, in which any of the tips of the protrusion portions is not placed on a hair follicle so that the brain wave electrode can be reliably in contact with a scalp.

## BRIEF DESCRIPTION OF THE DRAWINGS

Fig. 1 is a view schematically showing a brain wave measuring device according to an embodiment, in a state of being mounted on a human head.
Fig. 2 is a perspective view of a frame according to an embodiment.
Fig. 3 is a front view of a brain wave electrode unit according to an embodiment.
Fig. 4 is a perspective view of the brain wave detection electrode according to the embodiment.
Fig. 5 is a plan view of the brain wave detection electrode according to the embodiment.
Fig. 6 is a cross-sectional view of the brain wave detection electrode according to the embodiment.

Fig. 7 is a view showing a relationship between vertices of protrusion portions of the brain wave detection electrode and an arrangement of hair follicles according to an embodiment.

Fig. 8 is a view showing the relationship between vertices of protrusion portions of the brain wave detection electrode and an arrangement of hair follicles according to the embodiment.

Fig. 9 is a graph for describing a distance between vertices according to the embodiment.

Fig. 10 is plan view showing a variation of the protrusion portions according to the embodiment.

DESCRIPTION OF EMBODIMENTS

<Outline>

Hereinafter, embodiments of the present invention will be described with reference to the drawings.

Fig. 1 is a view schematically showing a brain wave measuring device 1 in a state of being mounted on a human head 99. A brain wave measuring method of mounting the brain wave measuring device 1 on the head 99 of a subject to measure brain waves is executed. The brain wave measuring device 1 is mounted on the head 99, detects brain waves as a potential fluctuation from a living body, and outputs the detected brain waves to a brain wave display device (not shown). The brain wave display device acquires the brain waves detected by the brain wave measuring device 1, and performs monitor display, data storage, and a well-known brain wave analysis process.

<Structure of brain wave measuring device 1>

As shown in Fig. 1, the brain wave measuring device 1 has a plurality of brain wave electrode units 10 and a frame 20. In the present embodiment, the brain wave electrode units 10 are provided for five channels (five units).

<Structure of frame 20>

Fig. 2 is a perspective view of the frame 20. The frame 20 is formed of a hard member such as a polyamide resin in a strip shape and is curved to follow a shape of the human head 99.

The frame 20 is provided with five electrode unit attachment portions 21 as openings for attaching the brain wave electrode units 10. A position of the electrode unit attachment portion 21 (that is, an attachment position of the brain wave electrode unit 10) corresponds to positions of T3, C3, Cz, C4, and T4 according to International 10-20 electrode placement method.

An inner peripheral surface of the electrode unit attachment portion 21 is threaded, and the brain wave electrode unit 10 is screwed by a helical part 13 of a body portion 11 (refer to Fig. 3). The amount of protrusion of the brain wave electrode unit 10 to the head 99 side is adjusted by adjusting the amount of screwing, and the contact amount and contact pressure with the head 99 (scalp) are controlled. In addition, the hair is combed by the operation of screwing the brain wave electrode unit 10.

<Structure of brain wave electrode unit 10>

Fig. 3 shows a front view of the brain wave electrode unit 10. The brain wave electrode unit 10 has a substantially columnar body portion 11 and a brain wave detection electrode 50 provided on one end side (lower side in the drawing) of the body portion 11.

The body portion 11 integrally has a signal output part 12, a helical part 13, and an electrode fixing part 14.

The helical part 13 has a shape helically formed on a side surface having a cylindrical shape. The signal output part 12 is provided at one end (upper side in the drawing) of the helical part 13. A signal output terminal is provided in the signal output part 12, and in a case where the brain wave electrode unit 10 is screwed to the frame 20, the brain wave electrode unit 10 is operated by an operator using a predetermined jig as necessary. A columnar electrode fixing part 14 is provided at the other end (lower side in the drawing) of the helical part 13. The brain wave detection electrode 50 is attached to the electrode fixing part 14.

<Structure of brain wave detection electrode 50>

Fig. 4 is a perspective view of the brain wave detection electrode 50. Fig. 5 is a plan view of the brain wave detection electrode 50. Fig. 6 is a cross-sectional view of the brain wave detection electrode 50, and particularly shows a cross-sectional view taken along a line X1-X1 of Fig. 5.

The brain wave detection electrode 50 has a base portion 51, a protrusion portion 60, and an electrode portion 80. The base portion 51 and the protrusion portion 60 are integrally provided by a rubber-like elastic body. A specific

material of the elastic body will be described later. The base portion 51 and the protrusion portion 60 are not limited to the configuration in which they are integrally provided, and may be configured by assembling those provided separately by an adhesive or a fitting structure.

The base portion 51 has a substantially columnar shape, and one end thereof is a protrusion forming surface 52 having a circular shape and the other end thereof is an attachment surface 53 having a circular shape. The attachment surface 53 is attached to the electrode fixing part 14 of the body portion 11 by an adhesive or the like. A fixing structure of the attachment surface 53 and the electrode fixing part 14 is not particularly limited, and for example, a fitting structure by an uneven shape may be used.

<Protrusion portion 60>

A plurality of the protrusion portions 60 are provided on the protrusion forming surface 52 in an aligned manner. Here, seven regular hexagonal pyramid protrusion portions 60 are arranged in a lattice shape. Specifically, the protrusion portions 60 are provided in a rhombic lattice shape. More specifically, as shown in Fig. 5, the six protrusion portions 60 are arranged in a honeycomb shape so as to surround the central protrusion portion 60 and so as to have no gap between bottom sides of protrusion portions 60 adjacent to each other. The electrode portion 80 is provided in range of a predetermined height from a vertex 61 in the protrusion portion 60, and comes into contact with the head 99 (scalp). A shape of the protrusion portion 60 may be a polygonal pyramid such as a triangular pyramid, a quadrangular pyramid, a heptagonal pyramid, a hexagonal pyramid, a heptagonal pyramid, and an octagonal pyramid, or a cone. In a case of the polygonal pyramid, an octagonal or lower pyramid is preferable, and a quadrangular pyramid or a hexagonal pyramid is more preferable.

In a case where the cone and the angular pyramid are compared as the protrusion portion 60, the angular pyramid has an advantage that a deformation direction is easily controlled as compared with the cone.

When the shape of the protrusion portion 60 is a quadrangular pyramid and a protrusion width and a protrusion height of the protrusion portion 60 are the same, in a case where the quadrangular pyramid and the hexagonal pyramid are compared as the shape of the protrusion portion 60, the hexagonal pyramid is less likely to be deformed than the quadrangular pyramid. Therefore, the projection width required to obtain the same strength is smaller in the hexagonal pyramid, and the number of projections which can be arranged on the protrusion forming surface 52 of the base portion 51, which is circular, can be increased.

In a case where the protrusion portion 60 is the quadrangular pyramid, an arrangement design of the plurality of the protrusion portions 60 is easy, and a deformation state when the protrusion portions 60 are pressed against the head 99 is easily predicted, so that stable brain wave measurement can be performed. Various shapes can be adopted as the lattice-shaped arrangement, and specific examples thereof will be described later with reference to Figs. 10A to 10E.

<Distance between protrusion portions 60 (vertices 61)>

Arrangement of the protrusion portions 60 will be described with particular attention to a relationship between a distance between the vertices 61 and hair follicles 9 with reference to Figs. 7 to 9. Figs. 7 and 8 are views showing the relationship between the vertices 61 of the protrusion portions 60 in the brain wave detection electrode and the arrangement of the hair follicles. In Fig. 7, the hair follicle 9 is shown by being added to the plan view of the brain wave detection electrode 50 (the electrode portion 80 is omitted). Fig. 8 is a view for facilitating the description of the relationship between the distance between the vertices 61, the gap between the hair follicles 9, and the size of the hair follicle 9.

As shown in Fig. 7, a distance D1 between protrusion portions 60 (that is, between vertices 61) most adjacent to each other satisfies the following expression (1).

$$D1 < L(m^2 + n^2)^{1/2} - d \text{ or } D1 > L(m^2 + n^2)^{1/2} + d \quad \ldots \quad (1)$$

m and n: an integer of 0 or more, in which either m or n is 1 or more
L: a distance between centers of hair follicles in the scalp of the subject
d: a diameter of the hair follicle in the scalp of the subject

The distance L between the centers of the hair follicles can be, for example, 0.5 mm or more and 1.5 mm or less, preferably 0.6 mm or more and 1.4 mm or less and more preferably 0.7 mm or more and 1.3 mm or less. In consideration of general tendency, the distance L between the centers of the hair follicles may be calculated as 1.0 mm.

The diameter d of the hair follicle can be, for example, 0.05 mm or more and 0.15 mm or less, preferably 0.06 mm or more and 0.14 mm or less and more preferably 0.07 mm or more and 0.13 mm or less. In consideration of general tendency, the diameter d of the hair follicle may be calculated as 0.1 mm.

The distance D1 between the protrusion portions 60 (that is, the vertices 61) is 2 mm or more and 10 mm or less, preferably 2.5 mm or more and 7 mm or less and more preferably 3 mm or more and 5 mm or less. By setting the distance D1 in such a range, the density of the protrusion portion 60 can be sufficiently increased, and the size or strength of the protrusion portion 60 can be appropriately set.

With reference to Fig. 8, five arrangement examples (arrangements (1) to (5)) will be described with a case where vertices 61 of two adjacent protrusion portions 60 are placed on the hair follicle 9 and a case of not being placed thereon. Here, a situation in which the vertices 61 of the protrusion portions 60 come into contact with the vicinity of a certain hair follicle 9, and the vicinity of an m-th hair follicle 9 in the right direction and an n-th hair follicle 9 in the upward direction with respect to the hair follicle 9 as a reference is assumed.

In the arrangement (1), the left vertex 61 in the drawing is located on the right side of the rightmost position of the left hair follicle 9. The right vertex 61 is located on the left side of the leftmost position of the right hair follicle 9. In this case, the distance D1 between the vertices 61 is shorter than a distance between the rightmost position of the left hair follicle 9 and the leftmost position of the right hair follicle 9, and satisfies the following expression (1A).

$$D1 < L(m^2 + n^2)^{1/2} - d \quad ... \quad (1A)$$

By adjusting the distance D1 between the vertices 61 to satisfy the above expression (1A), even in a case where the vertex 61 of one protrusion portion 60 is mounted on any portion of the hair follicle 9 (in a circle indicating the hair follicle 9 in the drawing), the vertex 61 of the other protrusion portion 60 is not mounted on the hair follicle 9.

In the arrangement (2), the left vertex 61 in the drawing is located at the rightmost position of the left hair follicle 9. The right vertex 61 is located at the leftmost position of the right hair follicle 9. In this case, the distance D1 between the vertices 61 is equal to a distance between the rightmost position of the left hair follicle 9 and the leftmost position of the right hair follicle 9, and satisfies the following expression (1B).

$$D1 = L(m^2 + n^2)^{1/2} - d \quad ... \quad (1B)$$

By adjusting the distance D1 between the vertices 61 to satisfy the above expression (1B), the vertices 61 of both protrusion portions 60 are mounted on the hair follicles 9 in a state of just the arrangement (2).

In the arrangement (3), the left vertex 61 in the drawing is located in a circle indicating the left hair follicle 9. The right vertex 61 is located in a circle indicating the right hair follicle 9. Here, for easy understanding, a state in which each vertex 61 is located at a center 9a of the hair follicle 9 is shown.

In this case, the distance D1 between the vertices 61 is equal to a distance between the centers 9a of the hair follicles 9, and satisfies the following expression (1C).

$$D1 = L(m^2 + n^2)^{1/2} \quad ... \quad (1C)$$

In the arrangement (4), the left vertex 61 in the drawing is located at the leftmost position of the left hair follicle 9. The right vertex 61 is located at the rightmost position of the right hair follicle 9. In this case, the distance D1 between the vertices 61 is equal to a distance between the leftmost position of the left hair follicle 9 and the rightmost position of the right hair follicle 9, and satisfies the following expression (1D) .

$$D1 = L(m^2 + n^2)^{1/2} + d \quad ... \quad (1D)$$

By adjusting the distance D1 between the vertices 61 to satisfy the above expression (1D), the vertices 61 of both protrusion portions 60 are mounted on the hair follicles 9 in a state of just the arrangement (4).

In the arrangement (5), the left vertex 61 in the drawing is located on the left side of the leftmost position of the left hair follicle 9. The right vertex 61 is located on the right side of the rightmost position of the right hair follicle 9. In this case, the distance D1 between the vertices 61 is longer than a distance between the leftmost position of the left hair follicle 9 and the rightmost position of the right hair follicle 9, and satisfies the following expression (1E).

$$D1 > L(m^2 + n^2)^{1/2} + d \quad ... \quad (1E)$$

By adjusting the distance D1 between the vertices 61 to satisfy the above expression (1E), even in a case

where the vertex 61 of one protrusion portion 60 is mounted on any portion of the hair follicle 9 (in a circle indicating the hair follicle 9 in the drawing), the vertex 61 of the other protrusion portion 60 is not mounted on the hair follicle 9.

From the above, both vertices 61 are mounted on the hair follicles 9 in the cases of the arrangements (2) to (4). That is, in a case where D1 satisfies the following expression (2) as the distance D1 between the vertices 61, both vertices 61 are mounted on the hair follicles 9.

$$L(m^2 + n^2)^{1/2} - d \leq D1 \leq L(m^2 + n^2)^{1/2} + d \quad \ldots \quad (2)$$

Therefore, in a case where the distance D1 between the vertices 61 is under a condition which does not satisfy the above expression (2), that is, in a case of under a condition which satisfies the above expression (1), it is possible to avoid that any of the vertices 61 of the two adjacent protrusion portions 60 is mounted on the hair follicles 9.

Fig. 9 is a graph showing the range of the distance D1 satisfying the above expression (1). Specifically, the range of the distance D1 is shown in a case where the set of (m, n) is sequentially applied with 1.0 mm of the distance L between the centers of the hair follicles and 0.1 mm of the diameter d of the hair follicle. Here, both m and n are 10 or less.

In the graph, the horizontal axis indicates the distance D1, and the vertical axis indicates n of (m, n). For example, in a case where n = 1 on the vertical axis, the range obtained from the expression (1) when m = 1, 2, 3, ..., and 10 are applied is shown as a horizontal line (bold line). The range satisfying the expression (1) for all the horizontal lines where n is 1 to 10, that is, for all the (m, n) combinations is a region indicated by the shaded area in the graph. In other words, in a case where the distance D1 corresponds to the shaded region, any of the vertices 61 of the two protrusion portions 60 is not mounted on the hair follicles 9.

Specifically, it is in a range shown in Table 1 below. However, the distance D1 is set to ≤ 10 mm.

[Table 1]

Table 1

| | |
|---|---|
| 0.10 < D < 0.90 | 5.49 < D < 5.56 |
| 1.10 < D < 1.31 | 6.18 < D < 6.22 |
| 1.51 < D < 1.90 | 6.50 < D < 6.61 |
| 2.10 < D < 2.14 | 6.81 < D < 6.90 |
| 2.34 < D < 2.73 | 7.38 < D < 7.52 |
| 3.26 < D < 3.51 | 8.35 < D < 8.39 |
| 3.71 < D < 3.90 | 8.70 < D < 8.84 |
| 4.34 < D < 4.37 | 9.32 < D < 9.33 |
| 4.57 < D < 4.90 | 9.59 < D < 9.75 |
| 5.20 < D < 5.29 | |

With reference to Figs. 10A to 10E, variations in the shape and arrangement of the protrusion portions 60 (vertices 61) will be described. In a brain wave detection electrode 50A shown in Fig. 10A, the protrusion portion 60 has a square pyramid shape. Nine protrusion portions 60 are arranged on the protrusion forming surface 52 in a 3 × 3 vertical and horizontal manner. That is, the protrusion portions 60 (vertices 61) are arranged in a square lattice shape.

In a brain wave detection electrode 50B shown in Fig. 10B, the protrusion portion 60 has a square pyramid shape. Seven protrusion portions 60 are arranged on the protrusion forming surface 52 in a rhombic lattice shape. Specifically, the protrusion portions 60 are arranged such that two protrusion portions are arranged in a first row, three protrusion portions are arranged in a second row, and two protrusion portions are arranged in a third row, so as to be vertically and horizontally symmetric, and such that protrusion portions 60 adjacent to each other are arranged so as to be in contact with each other without a gap at the bottom sides.

In a brain wave detection electrode 50C shown in Fig. 10C, the protrusion portion 60 has a cone shape. Nine protrusion portions 60 are arranged on the protrusion forming surface 52 in a 3 × 3 vertical and horizontal manner. That is, the protrusion portions 60 (vertices 61) are arranged in a square lattice shape.

In a brain wave detection electrode 50D shown in Fig. 10D, the protrusion portion 60 has a cone shape. Seven

protrusion portions 60 (vertices 61) are arranged in an equilateral triangle lattice on the protrusion forming surface 52, such that adjacent bottom sides are in contact with each other.

In a brain wave detection electrode 50E shown in Fig. 10E, the protrusion portion 60 has a regular triangular pyramid shape. Thirteen protrusion portions 60 are arranged in a triangular lattice shape on the protrusion forming surface 52, such that adjacent bottom sides overlap each other. The vertices 61 of the protrusion portions 60 are arranged in a regular hexagonal lattice shape.

As the shape and arrangement of the protrusion portions 60, various shapes can be adopted, but in the protrusion portions 60 arranged regularly, the distance D1 between the vertices 61 satisfies the above expression (1). Therefore, it is possible to avoid all the vertices 61, that is, all the electrode portions 80 from being reliably mounted on the hair follicles 9, and to realize stable brain wave measurement.

<Structure of signal line 69>

As shown in the cross-sectional shape of the brain wave detection electrode 50 of Fig. 6, a conductive signal line 69 connected to the electrode portion 80 is provided inside the protrusion portion 60. As the signal line 69, various arrangement structures can be adopted as long as the inside of the protrusion portion 60 is electrically connected. For example, a tip of the signal line 69 may have a structure which protrudes with respect to the tip of the protrusion portion 60 or an inclined surface of the tip portion of the protrusion portion 60, a structure which is substantially on the same plane, or a structure which is buried. From the viewpoint of connection stability with the electrode portion 80, a protruding structure may be used. A part or the entirety of the protruding portion of the tip of the signal line 69 is covered with the electrode portion 80.

As the protruding structure of the tip of the signal line 69, a structure without folding, a structure with folding, or a structure in which the signal line 69 is wound around the surface of the tip of the protrusion portion 60 may be adopted. In addition, the signal line 69 may be inclined with respect to the perpendicular line extending from the vertex 61 of the protrusion portion 60 without coinciding with the perpendicular line.

<Material of brain wave detection electrode 50 (base portion 51 and protrusion portion 60)>

A material of the brain wave detection electrode 50 (base portion 51 and protrusion portion 60) will be described. The brain wave detection electrode 50 is a rubber-like elastic body as described above. Specifically, the rubber-like elastic body is a rubber or a thermoplastic elastomer (also simply referred to as "elastomer (TPE)"). Examples of the rubber include a silicone rubber. Examples of the thermoplastic elastomer include styrene-based TPE (TPS), olefin-based TPE (TPO), vinyl chloride-based TPE (TPVC), urethane-based TPE (TPU), ester-based TPE (TPEE), and amide-based TPE (TPAE).

In a case where the brain wave detection electrode 50 is made of silicone rubber, a rubber hardness A is, for example, 15 or more and 55 or less in a case where a type A durometer hardness of the surface of the brain wave detection electrode 50 (the protrusion portion 60 or the base portion 51) is measured at 37°C in accordance with JIS K 6253 (1997) and is defined as the rubber hardness A.

Here, the above-described silicone rubber-based curable composition will be described.

The above-described silicone rubber can be formed of a cured product of the silicone rubber-based curable composition. A curing step of the silicone rubber-based curable resin composition is performed by heating (primary curing) the composition, for example, at 100°C to 250°C for 1 to 30 minutes and post-baking (secondary curing) the heated composition at 100°C to 200°C for 1 to 4 hours.

An insulating silicone rubber is a silicone rubber not containing a conductive filler, and a conductive silicone rubber is a silicone rubber containing a conductive filler.

The silicone rubber-based curable composition according to the present embodiment may contain a vinyl group-containing organopolysiloxane (A). The vinyl group-containing organopolysiloxane (A) is a polymer including the silicone rubber-based curable composition according to the present embodiment as a main component.

The insulating silicone rubber-based curable composition and the conductive silicone rubber-based curable composition may contain the same kind of vinyl group-containing linear organopolysiloxane. It is sufficient that the same kind of vinyl group-containing linear organopolysiloxane includes at least a vinyl group having the same functional group and is linear, and it may have different vinyl group amounts or molecular weight distributions in the molecule, or different addition amounts thereof.

The insulating silicone rubber-based curable composition and the conductive silicone rubber-based curable composition may contain different kinds of vinyl group-containing organopolysiloxanes.

The above-described vinyl group-containing organopolysiloxane (A) may include a vinyl group-containing linear organopolysiloxane (A1) having a linear structure.

The above-described vinyl group-containing linear organopolysiloxane (A1) has a linear structure and contains

a vinyl group, in which the vinyl group functions as a crosslinking point during the curing.

A content of the vinyl group in the vinyl group-containing linear organopolysiloxane (A1) is not particularly limited, and for example, the vinyl group-containing linear organopolysiloxane (A1) has two or more vinyl groups in the molecule, and the content thereof is preferably 15 mol% or less and more preferably 0.01 to 12 mol%. As a result, the amount of the vinyl group in the vinyl group-containing linear organopolysiloxane (A1) can be optimized, and a network between respective components described later can be reliably formed. In the present embodiment, a range represented by "to" includes numerical values of both ends.

In the present specification, the content of the vinyl group represents mol% of a vinyl group-containing siloxane unit with respect to 100 mol% of all units forming the vinyl group-containing linear organopolysiloxane (A1). However, it is assumed that one vinyl group is present for each vinyl group-containing siloxane unit.

In addition, a polymerization degree of the vinyl group-containing linear organopolysiloxane (A1) is not particularly limited, and is, for example, preferably in a range of approximately 1,000 to 10,000 and more preferably in a range of approximately 2,000 to 5,000. The polymerization degree can be obtained as, for example, a number-average polymerization degree (number-average molecular weight) in terms of polystyrene in gel permeation chromatography (GPC) in which chloroform is used as an eluent.

Furthermore, a specific gravity of the vinyl group-containing linear organopolysiloxane (A1) is not particularly limited and is preferably in a range of approximately 0.9 to 1.1.

By using the vinyl group-containing linear organopolysiloxane (A1) having the polymerization degree and the specific gravity within the above-described ranges, heat resistance, flame retardancy, chemical stability, and the like of the obtained silicone rubber can be improved.

It is preferable that the vinyl group-containing linear organopolysiloxane (A1) has a structure represented by Formula (1) .

[Chem. 1]

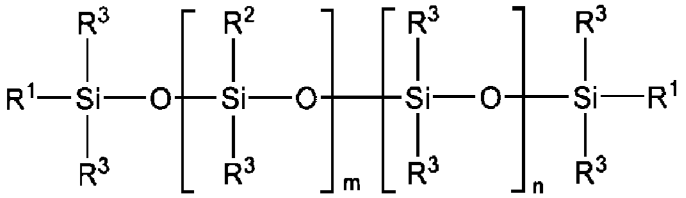

(1)

In Formula (1), $R^1$ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group, and among these, a methyl group is preferable. Examples of the alkenyl group having 1 to 10 carbon atoms include a vinyl group, an allyl group, and a butenyl group, and among these, a vinyl group is preferable. Examples of the aryl group having 1 to 10 carbon atoms include a phenyl group.

In addition, $R^2$ represents a substituted or unsubstituted alkyl group, alkenyl group, or aryl group having 1 to 10 carbon atoms, or a hydrocarbon group including a combination thereof. Examples of the alkyl group having 1 to 10 carbon atoms include a methyl group, an ethyl group, and a propyl group, and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, $R^3$ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group, and among these, a methyl group is preferable. Examples of the aryl group having 1 to 8 carbon atoms include a phenyl group.

Furthermore, in Formula (1), examples of a substituent of $R^1$ and $R^2$ include a methyl group and a vinyl group, and examples of a substituent of $R^3$ include a methyl group.

In Formula (1), a plurality of $R^1$'s may be independent from each other, and may be the same or different from each other. Furthermore, the same can be applied to $R^2$ and $R^3$.

Furthermore, m and n each independently represent the number of repeating units forming the vinyl group-containing linear organopolysiloxane (A1) represented by Formula (1), and m represents an integer of 0 to 2,000 and n represents an integer of 1,000 to 10,000. m preferably represents 0 to 1,000, and n preferably represents 2,000 to 5,000.

In addition, examples of a specific structure of the vinyl group-containing linear organopolysiloxane (A1) represented by Formula (1) include a structure represented by Formula (1-1).

[Chem. 2]

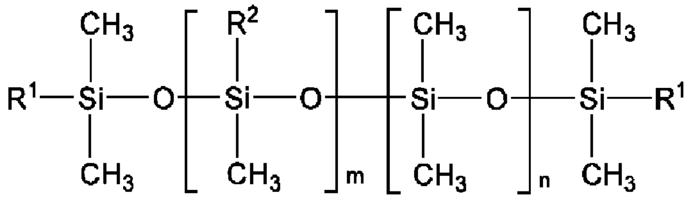

(1 − 1)

In Formula (1-1), $R^1$ and $R^2$ each independently represent a methyl group or a vinyl group, and at least one thereof represents a vinyl group.

Furthermore, as the vinyl group-containing linear organopolysiloxane (A1), it is preferable to contain a first vinyl group-containing linear organopolysiloxane (A1-1) having two or more vinyl groups in the molecule, in which the content of the vinyl group is 0.4 mol% or less, and a second vinyl group-containing linear organopolysiloxane (A1-2) in which the content of the vinyl group is 0.5 to 15 mol%. By using the first vinyl group-containing linear organopolysiloxane (A1-1) having the general content of the vinyl group and the second vinyl group-containing linear organopolysiloxane (A1-2) having the high content of the vinyl group in combination as raw rubber which is a raw material of the silicone rubber, the vinyl groups can be distributed, and a structure with high crosslinking density in the crosslinked network of the silicone rubber can be more effectively formed. As a result, a tearing strength of the silicone rubber can be more effectively improved.

Specifically, as the vinyl group-containing linear organopolysiloxane (A1), for example, it is preferable to use the first vinyl group-containing linear organopolysiloxane (A1-1) in which, in Formula (1-1) above, two or more units of an unit in which $R^1$ is a vinyl group and/or an unit in which $R^2$ is a vinyl group are included in the molecule and the content of the units is 0.4 mol% or less, and the second vinyl group-containing linear organopolysiloxane (A1-2) in which the content of the unit in which $R^1$ is a vinyl group and/or the unit in which $R^2$ is 0.5 to 15 mol%.

In addition, in the first vinyl group-containing linear organopolysiloxane (A1-1), the content of the vinyl group is preferably 0.01 to 0.2 mol%. In addition, in the second vinyl group-containing linear organopolysiloxane (A1-2), the content of the vinyl group is preferably 0.8 to 12 mol%.

Furthermore, in a case where the first vinyl group-containing linear organopolysiloxane (A1-1) and the second vinyl group-containing linear organopolysiloxane (A1-2) are mixed in combination, a ratio between (A1-1) and (A1-2) is not particularly limited, but a weight ratio (A1-1):(A1-2) is preferably 50:50 to 95:5 and more preferably 80:20 to 90:10.

As each of the first vinyl group-containing linear organopolysiloxane (A1-1) and the second vinyl group-containing linear organopolysiloxane (A1-2), only one kind may be used or two or more kinds may be used in combination.

In addition, the vinyl group-containing organopolysiloxane (A) may include a vinyl group-containing branched organopolysiloxane (A2) having a branched structure.

<<Organohydrogen polysiloxane (B)>>

The silicone rubber-based curable composition according to the present embodiment may contain a crosslinking agent. The crosslinking agent may include an organohydrogen polysiloxane (B).

The organohydrogen polysiloxane (B) is classified into a linear organohydrogen polysiloxane (B1) having a linear structure and a branched organohydrogen polysiloxane (B2) having a branched structure, and may include either or both of (B1) and (B2).

The insulating silicone rubber-based curable composition and the conductive silicone rubber-based curable composition may contain the same kind of crosslinking agent. It is sufficient that the same kind of crosslinking agent has at least a common structure such as a linear structure or a branched structure, and it may have different molecular weight distributions in the molecule, different functional groups, or different addition amounts thereof.

The insulating silicone rubber-based curable composition and the conductive silicone rubber-based curable composition may further contain different kinds of crosslinking agents.

The linear organohydrogen polysiloxane (B1) is a polymer that has a linear structure and a structure in which hydrogen is directly bonded to Si (=Si-H), and that is obtained by a hydrosilylation reaction of the vinyl group in the vinyl group-containing organopolysiloxane (A) and a vinyl group in a component mixed in the silicone rubber-based curable composition to crosslink the components.

A molecular weight of the linear organohydrogen polysiloxane (B1) is not particularly limited, and for example, a weight-average molecular weight thereof is preferably 20,000 or less and more preferably 1,000 or more and 10,000 or less.

A weight-average molecular weight of the linear organohydrogen polysiloxane (B1) can be measured in terms

of polystyrene in gel permeation chromatography (GPC) in which chloroform is used as an eluent.

In addition, it is preferable that the linear organohydrogen polysiloxane (B1) does not have a vinyl group typically. As a result, the crosslinking reaction in the molecule of the linear organohydrogen polysiloxane (B1) can be reliably prevented from progressing.

As such a linear organohydrogen polysiloxane (B1), for example, it is preferable to use an organohydrogen polysiloxane having a structure represented by Formula (2).

[Chem. 3]

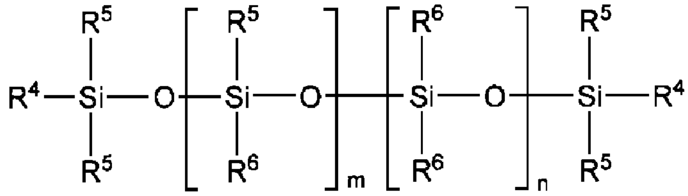

(2)

In Formula (2), $R^4$ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group, and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In addition, $R^3$ represents a substituted or unsubstituted alkyl group, alkenyl group, or aryl group having 1 to 10 carbon atoms, a hydrocarbon group including a combination thereof, or a hydride group. Examples of the alkyl group having 1 to 10 carbon atoms include a methyl group, an ethyl group, and a propyl group, and among these, a methyl group is preferable. Examples of the alkenyl group having 1 to 10 carbon atoms include a vinyl group, an allyl group, and a butenyl group. Examples of the aryl group having 1 to 10 carbon atoms include a phenyl group.

In Formula (2), a plurality of $R^4$'s may be independent from each other, and may be the same or different from each other. The same can be applied to $R^5$. In this case, at least two or more of a plurality of $R^4$'s and $R^5$'s represent a hydride group.

In addition, $R^6$ represents a substituted or unsubstituted alkyl group or aryl group having 1 to 8 carbon atoms, or a hydrocarbon group including a combination thereof. Examples of the alkyl group having 1 to 8 carbon atoms include a methyl group, an ethyl group, and a propyl group, and among these, a methyl group is preferable. Examples of the aryl group having 1 to 8 carbon atoms include a phenyl group. A plurality of $R^6$'s may be independent from each other, and may be the same or different from each other.

Examples of a substituent of $R^4$, $R^5$, and $R^6$ in Formula (2) include a methyl group and a vinyl group, and from the viewpoint of preventing the crosslinking reaction in the molecule, a methyl group is preferable.

Furthermore, m and n each independently represent the number of repeating units forming the linear organohydrogen polysiloxane (B1) represented by Formula (2), and m represents an integer of 2 to 150 and n represents an integer of 2 to 150. It is preferable that m represents an integer of 2 to 100 and n represents an integer of 2 to 100.

As the linear organohydrogen polysiloxane (B1), only one kind may be used alone, or two or more kinds may be used in combination.

Since the branched organohydrogen polysiloxane (B2) has a branched structure, it is a component that largely contributes to a formation of a structure with high crosslinking density in the silicone rubber system by forming a region having a high crosslinking density. In addition, same as the above-described linear organohydrogen polysiloxane (B1), the branched organohydrogen polysiloxane (B2) is a polymer that has a structure in which hydrogen is directly bonded to Si (=Si-H), and that is obtained by a hydrosilylation reaction of the vinyl group in the vinyl group-containing organopolysiloxane (A) and a vinyl group in a component mixed in the silicone rubber-based curable composition to crosslink the components.

In addition, a specific gravity of the branched organohydrogen polysiloxane (B2) is in a range of 0.9 to 0.95.

Furthermore, it is preferable that the branched organohydrogen polysiloxane (B2) does not have a vinyl group typically. As a result, the crosslinking reaction in the molecule of the branched organohydrogen polysiloxane (B2) can be reliably prevented from progressing.

In addition, it is preferable that the branched organohydrogen polysiloxane (B2) is represented by Average Compositional Formula (c) below.

Average Compositional Formula (c)

$$(H_a(R^7)_{3-a}SiO_{1/2})_m(SiO_{4/2})_n$$

(in Formula (c), $R^7$ represents a monovalent organic group, a represents an integer in a range of 1 to 3, m represents the number of $H_a(R^7)_{3-a}SiO_{1/2}$ units, and n represents the number of $SiO_{4/2}$ units)

In Formula (c), $R^7$ represents a monovalent organic group, and preferably represents a substituted or unsubstituted alkyl group or aryl group having 1 to 10 carbon atoms, or a hydrocarbon group including a combination thereof. Examples of the alkyl group having 1 to 10 carbon atoms include a methyl group, an ethyl group, and a propyl group, and among these, a methyl group is preferable. Examples of the aryl group having 1 to 10 carbon atoms include a phenyl group.

In Formula (c), a represents the number of hydride groups (hydrogen atoms directly bonded to Si), which is an integer in a range of 1 to 3 and preferably 1.

In addition, in Formula (c), m represents the number of $H_a(R^7)_{3-a}SiO_{1/2}$ units, and n represents the number of $SiO_{4/2}$ units.

The branched organohydrogen polysiloxane (B2) has a branched structure. The linear organohydrogen polysiloxane (B1) and the branched organohydrogen polysiloxane (B2) are different from each other in that whether the structure is linear or branched. The number (R/Si) of alkyl groups R bonded to Si with respect to the number of Si, which is set to 1, is 1.8 to 2.1 in the linear organohydrogen polysiloxane (B1) and is 0.8 to 1.7 in the branched organohydrogen polysiloxane (B2).

Since the branched organohydrogen polysiloxane (B2) has a branched structure, an amount of residues is 5% or more, for example, after heating to 1,000°C at a temperature increase rate of 10 °C/min in a nitrogen atmosphere. On the other hand, since the linear organohydrogen polysiloxane (B1) is linear, an amount of residues after the heating under the above-described conditions is substantially 0.

In addition, specific examples of the branched organohydrogen polysiloxane (B2) include an organohydrogen polysiloxane having a structure represented by Formula (3).

[Chem. 4]

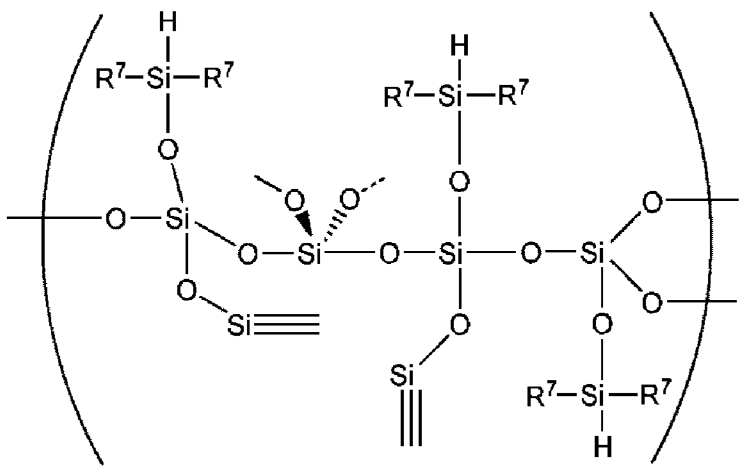

(3)

In Formula (3), $R^7$ represents a substituted or unsubstituted alkyl group or aryl group having 1 to 8 carbon atoms, a hydrocarbon group including a combination thereof, or a hydrogen atom. Examples of the alkyl group having 1 to 8 carbon atoms include a methyl group, an ethyl group, and a propyl group, and among these, a methyl group is preferable. Examples of the aryl group having 1 to 8 carbon atoms include a phenyl group. Examples of a substituent of $R^7$ include a methyl group.

In Formula (3), a plurality of $R^7$'s may be independent from each other, and may be the same or different from each other.

In addition, in Formula (3), "-O-Si=" represents that Si has a branched structure which spreads three-dimensionally.

As the branched organohydrogen polysiloxane (B2), only one kind may be used alone, or two or more kinds may be used in combination.

In addition, in each of the linear organohydrogen polysiloxane (B1) and the branched organohydrogen polysiloxane (B2), an amount of hydrogen atoms directly bonded to Si (hydride groups) is not particularly limited. However, in the silicone rubber-based curable composition, the total amount of hydride groups in the linear organohydrogen

polysiloxane (B1) and the branched organohydrogen polysiloxane (B2) is preferably 0.5 to 5 mol and more preferably 1 to 3.5 mol with respect to 1 mol of vinyl groups in the vinyl group-containing linear organopolysiloxane (A1). As a result, a crosslinked network can be reliably formed between the linear organohydrogen polysiloxane (B1) and the branched organohydrogen polysiloxane (B2), and the vinyl group-containing linear organopolysiloxane (A1).

<<Silica particles (C)>>

The silicone rubber-based curable composition according to the present embodiment contains a non-conductive filler. The non-conductive filler may include silica particles (C) as necessary. As a result, hardness or mechanical strength of the elastomer can be improved.

The insulating silicone rubber-based curable composition and the conductive silicone rubber-based curable composition may contain the same kind of non-conductive filler. It is sufficient that the same kind of non-conductive filler may have at least a common constituent material, and the particle diameter, the specific surface area, the surface treatment agent, or the amount added may be different.

The insulating silicone rubber-based curable composition and the conductive silicone rubber-based curable composition may further contain different kinds of silane coupling agents.

The silica particles (C) are not particularly limited, and for example, fumed silica, pyrogenic silica, or precipitated silica is used. These may be used alone or in combination of two or more thereof.

A specific surface area of the silica particles (C), which measured by, for example, a BET method, is, for example, preferably 50 to 400 $m^2/g$ and more preferably 100 to 400 $m^2/g$. In addition, an average primary particle size of the silica particles (C) is, for example, preferably 1 to 100 nm and more preferably approximately 5 to 20 nm.

By using the silica particles (C) having the specific surface area and the average particle size within the above-described range, the hardness or mechanical strength, in particular, the tensile strength of the formed silicone rubber can be improved.

<<Silane coupling agent (D)>>

The silicone rubber-based curable composition according to the present embodiment may contain a silane coupling agent (D).

The silane coupling agent (D) may have a hydrolyzable group. The hydrolyzable group is hydrolyzed into a hydroxyl group by water, this hydroxyl group reacts with a hydroxyl group on a surface of the silica particles (C) in a dehydration synthesis reaction, and as a result, the surface of the silica particles (C) can be modified.

The insulating silicone rubber-based curable composition and the conductive silicone rubber-based curable composition may contain the same kind of silane coupling agent. It is sufficient that the same kind of silane coupling agent has at least a common functional group, and it may have different functional groups in the molecule or different addition amounts thereof.

The insulating silicone rubber-based curable composition and the conductive silicone rubber-based curable composition may further contain different kinds of silane coupling agents.

In addition, the silane coupling agent (D) may include a silane coupling agent having a hydrophobic group. As a result, the hydrophobic group is added to a surface of the silica particles (C), and in the silicone rubber-based curable composition and in the silicone rubber, it is presumed that a cohesion force between the silica particles (C) decreases (cohesion through a hydrogen bond formed by a silanol group decreases), and thus dispersibility of the silica particles (C) in the silicone rubber-based curable composition is improved. Accordingly, an interface between the silica particles (C) and a rubber matrix increases, and a reinforcing effect of the silica particles (C) increases. Furthermore, it is presumed that, when the rubber matrix is deformed, slipperiness of the silica particles (C) in the matrix is improved. By improving the dispersibility and slipperiness of the silica particles (C), a mechanical strength (for example, a tensile strength, a tearing strength, or the like) of the silicone rubber by the silica particles (C) is improved.

Furthermore, the silane coupling agent (D) may include a silane coupling agent having a vinyl group. As a result, the vinyl group is introduced into the surface of the silica particles (C). Therefore, when the silicone rubber-based curable composition is cured, that is, when the vinyl group in the vinyl group-containing organopolysiloxane (A) and the hydride group in the organohydrogen polysiloxane (B) react with each other in a hydrosilylation reaction such that a network (crosslinked structure) is formed, the vinyl group in the silica particles (C) gets involved with the hydrosilylation reaction with the hydride group in the organohydrogen polysiloxane (B). Accordingly, the silica particles (C) are also incorporated into the network. As a result, low hardness and high modulus of the formed silicone rubber can be achieved.

As the silane coupling agent (D), the silane coupling agent having a hydrophobic group and the silane coupling agent having a vinyl group can be used in combination.

Examples of the silane coupling agent (D) include a silane coupling agent represented by Formula (4).

$$Y_n\text{-Si-}(X)_{4-n} \cdots \qquad (4)$$

In Formula (4), n represents an integer of 1 to 3. Y represents any functional group of a hydrophobic group, a hydrophilic group, or a vinyl group, in a case where n represents 1, Y represents a hydrophobic group, and in a case where n represents 2 or 3, at least one of Y's represents a hydrophobic group. X represents a hydrolyzable group.

The hydrophobic group is an alkyl group or aryl group having 1 to 6 carbon atoms, or a hydrocarbon group including a combination thereof. Examples thereof include a methyl group, an ethyl group, a propyl group, and a phenyl group, and among these, a methyl group is particularly preferable.

In addition, examples of the hydrophilic group include a hydroxyl group, a sulfonate group, a carboxyl group, and a carbonyl group, and among these, a hydroxyl group is particularly preferable. The hydrophilic group may be included as a functional group, but from the viewpoint of imparting hydrophobicity to the silane coupling agent (D), it is preferable that the hydrophilic group is not included.

Furthermore, examples of the hydrolyzable group include an alkoxy group such as a methoxy group and an ethoxy group, a chloro group, and a silazane group, and among these, from the viewpoint of high reactivity with the silica particles (C), a silazane group is preferable. A silane coupling agent having a silazane group as the hydrolyzable group has a structure including two structures represented by ($Y_n$-Si-) in Formula (4) above.

Specific examples of the silane coupling agent (D) represented by Formula (4) are as follows.

Examples of the silane coupling agent having a hydrophobic group as the functional group include an alkoxysilane such as methyltrimethoxysilane, dimethyldimethoxysilane, phenyltrimethoxysilane, methyltriethoxysilane, dimethyldiethoxysilane, phenyltriethoxysilane, n-propyltrimethoxysilane, n-propyltriethoxysilane, hexyltrimethoxysilane, hexyltriethoxysilane, and decyltrimethoxysilane; a chlorosilane such as methyltrichlorosilane, dimethyldichlorosilane, trimethylchlorosilane, and phenyltrichlorosilane; and hexamethyldisilazane. Among these, a silane coupling agent having a trimethylsilyl group, which includes one or more selected from the group consisting of hexamethyldisilazane, trimethylchlorosilane, trimethylmethoxysilane, and trimethylethoxysilane, is preferable.

Examples of the silane coupling agent having a vinyl group as the functional group include an alkoxysilane such as methacryloxypropyltriethoxysilane, methacryloxypropyltrimethoxysilane, methacryloxypropylmethyldiethoxysilane, methacryloxypropylmethyldimethoxysilane, vinyltriethoxysilane, vinyltrimethoxysilane, and vinylmethyldimethoxysilane; a chlorosilane such as vinyltrichlorosilane and vinylmethyldichlorosilane; and divinyltetramethyldisilazane. Among these, a silane coupling agent having a vinyl group-containing organosilyl group, which includes one or more selected from the group consisting of methacryloxypropyltriethoxysilane, methacryloxypropyltrimethoxysilane, methacryloxypropylmethyldiethoxysilane, methacryloxypropylmethyldimethoxysilane, divinyltetramethyldisilazane, vinyltriethoxysilane, vinyltrimethoxysilane, and vinylmethyldimethoxysilane, is preferable.

In addition, in a case where the silane coupling agent (D) includes two kinds including the silane coupling agent having a trimethylsilyl group and the silane coupling agent having a vinyl group-containing organosilyl group, it is preferable that hexamethyldisilazane is included as the silane coupling agent having a hydrophobic group and divinyltetramethyldisilazane is included as the silane coupling agent having a vinyl group.

In a case where a silane coupling agent (D1) having a trimethylsilyl group and a silane coupling agent (D2) having a vinyl group-containing organosilyl group are used in combination, a ratio between (D1) and (D2) is not particularly limited, but a weight ratio (D1):(D2) is 1:0.001 to 1:0.35, preferably 1:0.01 to 1:0.20 and more preferably 1:0.03 to 1:0.15. By setting such a numerical range, desired physical properties of the silicone rubber can be obtained. Specifically, a balance between the dispersibility of silica in the rubber and the crosslinkability of the rubber can be achieved.

In the present embodiment, a lower limit value of a content of the silane coupling agent (D) is preferably 1 mass% or more, more preferably 3 mass% or more, and still more preferably 5 mass% or more with respect to 100 parts by weight of the total amount of the vinyl group-containing organopolysiloxane (A). In addition, an upper limit value of the content of the silane coupling agent (D) is preferably 100 mass% or less, more preferably 80 mass% or less, and still more preferably 40 mass% or less with respect to 100 parts by weight of the total amount of the vinyl group-containing organopolysiloxane (A).

By adjusting the content of the silane coupling agent (D) to be the above-described lower limit value or more, adhesiveness between a cylindrical portion containing an elastomer and a conductive resin layer can be improved. In addition, the improvement of the mechanical strength of the silicone rubber can be promoted. In addition, by adjusting the content of the silane coupling agent (D) to be the above-described upper limit value or less, the silicone rubber can have appropriate mechanical properties.

<<Platinum or platinum compound (E)>>

The silicone rubber-based curable composition according to the present embodiment may contain a catalyst. The catalyst may include a platinum or platinum compound (E). The platinum or platinum compound (E) is a catalyst component which functions as a catalyst during the curing. The addition amount of the platinum or platinum compound

(E) is the amount of the catalyst.

The insulating silicone rubber-based curable composition and the conductive silicone rubber-based curable composition may contain the same kind of catalyst. It is sufficient that the same kind of catalyst has at least a common constituent material, and it may have different compositions in the catalyst or different addition amounts thereof.

The insulating silicone rubber-based curable composition and the conductive silicone rubber-based curable composition may further contain different kinds of catalysts.

As the platinum or platinum compound (E), a well-known compound can be used, and examples thereof include platinum black, silica or carbon black on which platinum is supported, chloroplatinic acid or an alcohol solution of chloroplatinic acid, a complex salt of chloroplatinic acid and olefin, and a complex salt of chloroplatinic acid and vinylsilxoane.

As the platinum or platinum compound (E), only one kind may be used alone or two or more kinds may be used in combination.

In the present embodiment, a content of the platinum or platinum compound (E) in the silicone rubber-based curable composition refers to the amount of the catalyst and can be appropriately set. Specifically, a content of platinum-group metal in units of weight is 0.01 to 1000 ppm, preferably 0.1 to 500 ppm, with respect to 100 parts by weight of the total amount of the vinyl group-containing organopolysiloxane (A), the silica particles (C), and the silane coupling agent (D).

By adjusting the content of the platinum or platinum compound (E) to be the above-described lower limit value or more, the silicone rubber-based curable composition can be cured at an appropriate rate. In addition, by adjusting the content of the platinum or platinum compound (E) to be the above-described upper limit value or less, a reduction in manufacturing costs can be promoted.

<<Water (F)>>

In addition, the silicone rubber-based curable composition according to the present embodiment may contain water (F) in addition to the above-described components (A) to (E).

The water (F) is a component which functions as a dispersion medium for dispersing the respective components in the silicone rubber-based curable composition and contributes to the reaction between the silica particles (C) and the silane coupling agent (D). Therefore, in the silicone rubber, the silica particles (C) and the silane coupling agent (D) can be more reliably linked to each other, and uniform properties can be exhibited as a whole.

(Other components)

Furthermore, the silicone rubber-based curable composition according to the present embodiment may further contain other components in addition to the above-described components (A) to (F). Examples of the other components include an inorganic filler other than the silica particles (C), such as diatomaceous earth, iron oxide, zinc oxide, titanium oxide, barium oxide, magnesium oxide, cerium oxide, calcium carbonate, magnesium carbonate, zinc carbonate, glass wool, and mica; and an additive such as a reaction inhibitor, a dispersant, a pigment, a dye, an antistatic agent, an antioxidant, a flame retardant, and a thermal conductivity enhancing agent.

The conductive solution (conductive silicone rubber composition) according to the present embodiment contains the above-described conductive filler and a solvent, in addition to the above-described silicone rubber-based curable composition not containing the conductive filler.

As the above-described solvent, various well-known solvents can be used, and for example, a high boiling point solvent can be contained. These may be used alone or in combination of two or more thereof.

Examples of the solvent include an aliphatic hydrocarbon such as pentane, hexane, cyclohexane, heptane, methylcyclohexane, ethylcyclohexane, octane, decane, dodecane, and tetradecane; an aromatic hydrocarbon such as benzene, toluene, ethylbenzene, xylene, trifluoromethylbenzene, and benzotrifluoride; an ether such as diethyl ether, diisopropyl ether, dibutyl ether, cyclopentyl methyl ether, cyclopentyl ethyl ether, ethylene glycol dimethyl ether, ethylene glycol diethyl ether, diethylene glycol dimethyl ether, 1,4-dioxane, 1,3-dioxane, and tetrahydrofuran; a haloalkane such as dichloromethane, chloroform, 1,1-dichloroethane, 1,2-dichloroethane, 1,1,1-trichloroethane, and 1,1,2-trichloroethane; a carboxylic acid amid such as N,N-dimethylformamide and N,N-dimethylacetamide; and a sulfoxide such as dimethyl sulfoxide and diethyl sulfoxide. These may be used alone or in combination of two or more thereof.

By adjusting the solid content in the solution, the above-described conductive solution can have an appropriate viscosity for various coating methods such as spray coating and dip coating.

In addition, in a case where the above-described conductive solution contains the above-described conductive filler and the above-described silica particles (C), a lower limit value of a content of the silica particles (C) in the electrode portion 80 is, for example, 1 mass% or more, preferably 3 mass% or more and more preferably 5 mass% or more, with respect to 100 mass% of the total amount of the silica particles (C) and the conductive filler. As a result, the mechanical strength of the electrode 13 can be improved. On the other hand, an upper limit value of the above-described content

of the silica particles (C) in the electrode portion 80 is, for example, 20 mass% or less, preferably 15 mass% or less and more preferably 10 mass% or less, with respect to 100 mass% of the total amount of the silica particles (C) and the conductive filler. As a result, a balance between the conductivity and the mechanical strength or flexibility in the electrode portion 80 can be achieved.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the electrode portion 80 can be suppressed.

<Material of signal line 69>

As the signal line 69, a well-known material can be used, and for example, the signal line 69 can be formed of a conductive fiber. As the conductive fiber, one or more selected from the group consisting of metal fiber, metal-coated fiber, carbon fiber, conductive polymer fiber, conductive polymer-coated fiber, and conductive paste-coated fiber can be used. These may be used alone or in combination of two or more thereof.

A metal material of the metal fiber and the metal-coated fiber described above is not particularly limited as long as it has conductivity, and examples thereof include copper, silver, gold, nickel, tin, lead, zinc, bismuth, antimony, stainless steel, aluminum, and an alloy thereof. These may be used alone or in combination of two or more thereof. Among these, from the viewpoint of conductivity, silver can be used. In addition, it is preferable that the metal material does not include metal such as chromium, which imposes burden on the environment.

The fiber material of the metal-coated fiber, the conductive polymer-coated fiber, or the conductive paste-coated fiber described above is not particularly limited and may be any one of synthetic fiber, semisynthetic fiber, or natural fiber. Among these, for example, polyester, nylon, polyurethane, silk, or cotton is preferably used. These may be used alone or in combination of two or more thereof.

Examples of the above-described carbon fiber include a PAN-based carbon fiber and a pitch-based carbon fiber.

As a conductive polymer material of the conductive polymer fiber and the conductive polymer-coated fiber described above, for example, polythiophene, polypyrrole, polyaniline, polyacetylene, polyphenylene vinylene, polynaphthalene, a mixture of the conductive polymer and the binder resin, for example, a derivatives thereof, or an aqueous solution of the conductive polymer PEDOT-PSS ((3,4-ethylenedioxythiophene)-poly(styrene sulfonate)) is used.

A resin material in the conductive paste of the conductive paste-coated fiber described above is not particularly limited and preferably has elasticity. For example, the resin material includes one or more selected from the group consisting of silicone rubber, urethane rubber, fluorine rubber, nitrile rubber, acrylic rubber, styrene rubber, chloroprene rubber, and ethylene propylene rubber. These may be used alone or in combination of two or more thereof.

A conductive filler in the conductive paste of the conductive paste-coated fiber described above is not particularly limited, and a well-known conductive material may be used. For example, the conductive filler may include one or more selected from the group consisting of metal particles, metal fiber, metal-coated fiber, carbon black, acetylene black, graphite, carbon fiber, carbon nanotube, a conductive polymer, conductive polymer-coated fiber, and metal nanowire.

A metal forming the above-described conductive filler is not particularly limited. For example, the metal may include at least one or two or more among copper, silver, gold, nickel, tin, lead, zinc, bismuth, antimony, silver or silver chloride, and alloys thereof. Among these, from the viewpoint of high conductivity or high availability, silver or copper is preferable.

The above-described signal line 69 may be formed of twisted yarn obtained by twisting a plurality of linear conductive fibers. As a result, disconnection of the signal line 69 during deformation can be suppressed.

In the present embodiment, the coating of the conductive fiber includes not only that the outer surface of the fiber material is covered with the conductive fiber, but also that gaps between fibers in twisted yarn obtained by twisting single fibers are impregnated with metal, the conductive polymer, or the conductive paste such that each of the single fibers forming the twisted yarn is coated with the conductive fiber.

A tensile elongation at break of the signal line 69 is, for example, 1% or more and 50% or less, preferably 1.5% or more and 45%. In such a numerical range, excessive deformation of the protrusion portion 60 can be suppressed while suppressing break during deformation.

<Material of electrode portion 80>

The conductive member of the electrode portion 80 is, for example, a paste containing a highly conductive metal. The highly conductive metal includes one or more selected from the group consisting of copper, silver, gold, nickel, tin, lead, zinc, bismuth, antimony, and an alloy thereof. In particular, from the viewpoint of availability and conductivity, silver, silver chloride, or copper is preferable.

In a case where the electrode portion 80 is formed with the paste containing a highly conductive metal, the vertex 61 of the protrusion portion 60 made of the rubber-like elastic body is dipped (dip-coated) in a paste-like conductive

solution containing the highly conductive metal. Accordingly, the electrode portion 80 is formed on the surface of the protrusion portion 60.

The electrode portion 80 as a conductive resin layer may be formed by applying the conductive solution containing the conductive filler and the solvent onto the surface of the protrusion portion 60. At this time, the solvent is made of the same material (silicone rubber) as the material of the protrusion portion 60, so that the adhesiveness of the electrode portion 80 (conductive resin layer) can be enhanced.

By optionally heating and drying the conductive solution, the conductive silicone rubber is obtained.

The conductive silicone rubber may be configured not to contain a silicone oil. As a result, a decrease in conductivity due to bleeding out the silicone oil to a surface of the electrode portion 80 can be suppressed.

As a result, it is possible to improve the hair separating performance in a case where the brain wave measuring device 1 is mounted on the head 99. In addition, it is possible to sufficiently secure the contact area of the electrode portion 80 in a case where the brain wave measuring device 1 is mounted.

<Manufacturing method of brain wave detection electrode 50>

An example of a manufacturing method of the brain wave detection electrode 50 according to the present embodiment may include the following steps.

First, the above-described silicone rubber-based curable composition is molded by hot press molding using a mold to obtain a molded product including the base portion 51 and the protrusion portion 60. Subsequently, the signal line 69 is passed through the inside of each of the protrusion portions 60 of the obtained molded product using a sewing needle. Thereafter, a paste-like conductive solution is dip-coated on the protrusion portion 60 (a predetermined range including the vertex 61) of the obtained molded product, and post-curing is performed after heating and drying. Accordingly, the electrode portion 80 can be formed on the surface of the protrusion portion 60.

As a result, the brain wave detection electrode 50 can be manufactured.

In the above-described molding step, the above-described silicone rubber-based curable composition may be introduced into a molding space where the signal lines 69 are arranged such that insert molding of pressurizing and heating the composition is used.

The embodiments of the present invention have been described above, but these are examples of the present invention and various configurations other than the above can be adopted.

Priority is claimed on Japanese Patent Application No. 2021-155434, filed on September 24, 2021, the disclosure of which is incorporated herein by reference.

REFERENCE SIGNS LIST

| | |
|---|---|
| 1 | brain wave measuring device |
| 10 | brain wave electrode unit |
| 11 | body portion |
| 12 | signal output part |
| 13 | helical part |
| 14 | electrode fixing part |
| 20 | frame |
| 21 | electrode unit attachment portion |
| 50, 50A to 50E | brain wave detection electrode |
| 51 | base portion |
| 52 | protrusion forming surface |
| 60 | protrusion portion |
| 61 | vertex |
| 69 | signal line |
| 80 | electrode portion |

**Claims**

1. A brain wave detection electrode which is brought into contact with a scalp of a subject to detect brain waves, the brain wave detection electrode comprising:

a base portion;
a plurality of pyramid-shaped protrusion portions protruding from the base portion; and
an electrode portion provided in the protrusion portion,
wherein a distance D1 between protrusion portions most adjacent to each other among the plurality of protrusion portions satisfies the following expression (1),

$$D1 < L(m^2 + n^2)^{1/2} - d \text{ or } D1 > L(m^2 + n^2)^{1/2} + d \quad \ldots \quad (1)$$

m and n: an integer of 0 or more, in which either m or n is 1 or more,
L: a distance between centers of hair follicles in the scalp of the subject, and
d: a diameter of the hair follicle in the scalp of the subject.

**2.** The brain wave detection electrode according to claim 1, wherein the plurality of protrusion portions are arranged with a configuration in which vertices of the plurality of protrusion portions form a square lattice.

**3.** The brain wave detection electrode according to claim 1, wherein the plurality of protrusion portions are arranged with a configuration in which vertices of the plurality of protrusion portions form an equilateral triangle lattice.

**4.** The brain wave detection electrode according to claim 1 or 2, wherein the protrusion portion is a pyramid.

**5.** The brain wave detection electrode according to claim 4, wherein the pyramid of the protrusion portion is a quadrangular pyramid.

**6.** The brain wave detection electrode according to claim 5, wherein the protrusion portions are arranged such that sides of bottom surfaces of adjacent protrusion portions are in contact with each other.

**7.** The brain wave detection electrode according to claim 6, wherein the pyramid of the protrusion portion is a cone.

**8.** The brain wave detection electrode according to claim 1 or 2, wherein the distance D1 between the protrusion portions most adjacent to each other is 2 mm or more and 10 mm or less.

**9.** A brain wave measuring device comprising:

the brain wave detection electrode according to claim 1 or 2; and
a frame to which the brain wave detection electrode is attached, the frame being mounted on the head of the subject.

**10.** A brain wave measuring method comprising: mounting the brain wave measuring device according to claim 9 on the head of the subject and measuring brain waves.

FIG. 1

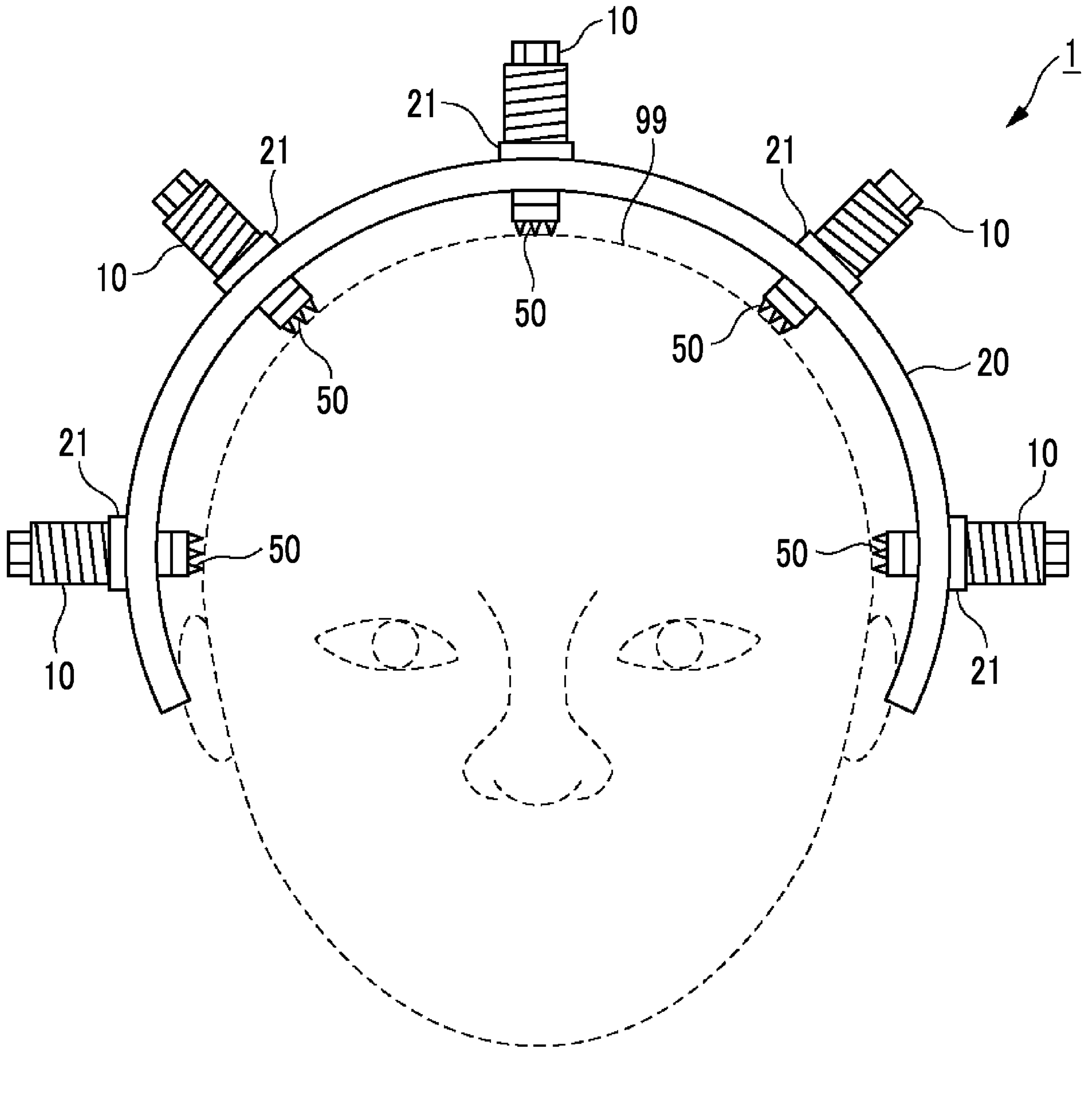

FIG. 2

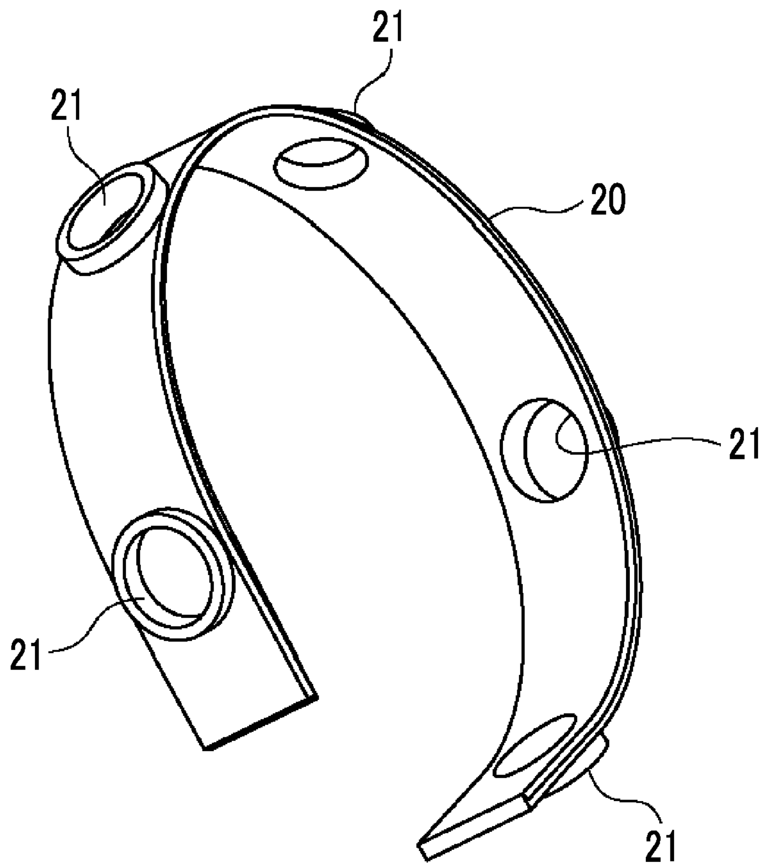

FIG. 3

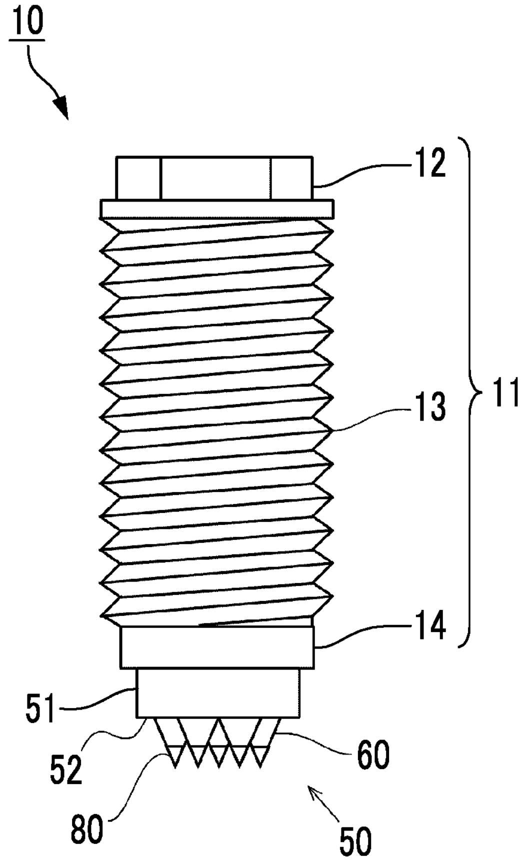


FIG. 4

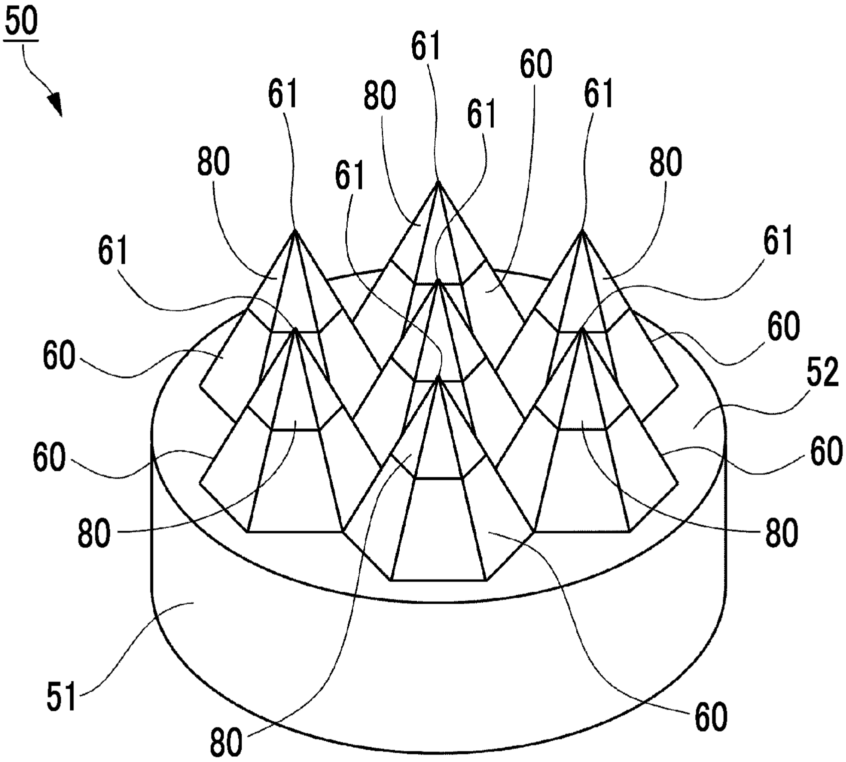

FIG. 5

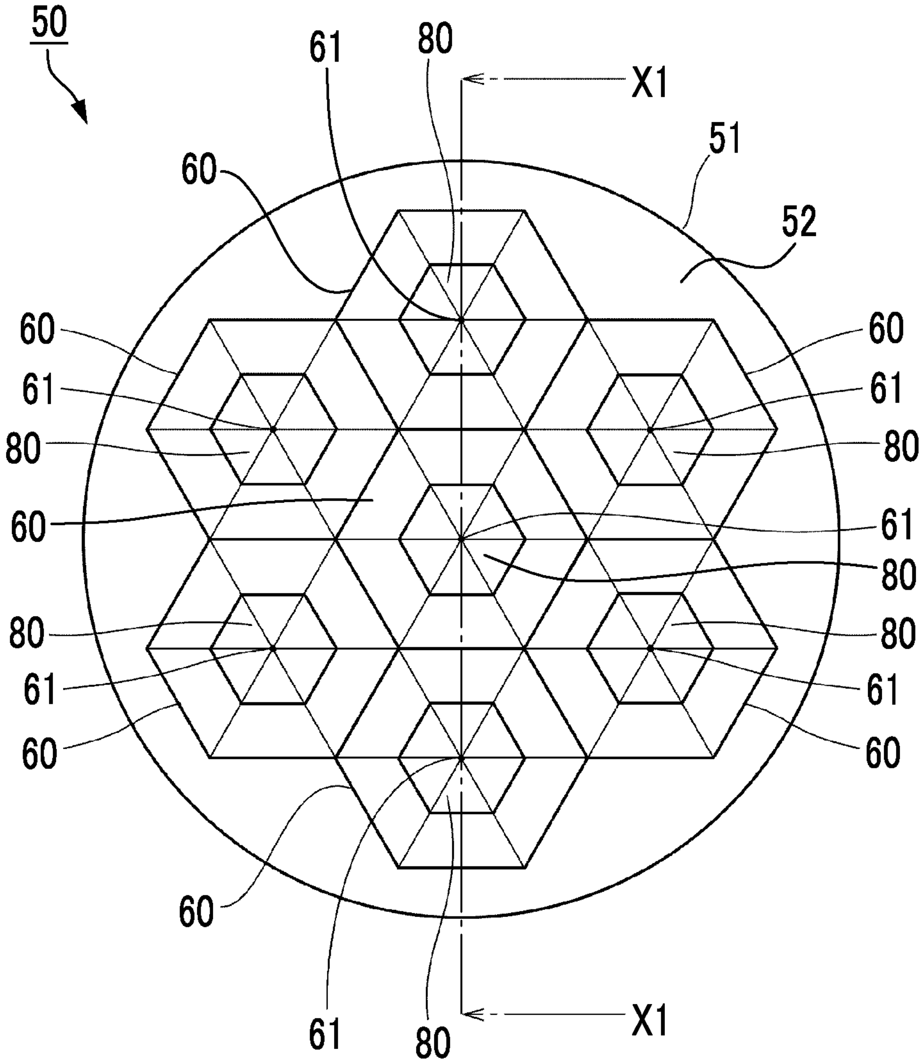
50
61
80
X1
60
51
52
60
60
61
61
80
80
60
61
80
80
80
61
61
60
60
60
61
80
X1

FIG. 6

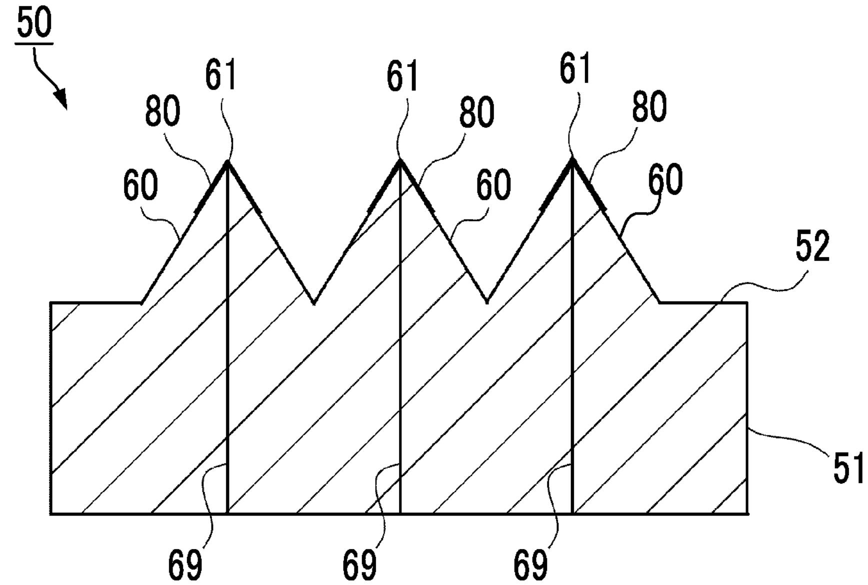
50
61
61
61
80
80
80
60
60
60
52
51
69
69
69

FIG. 7

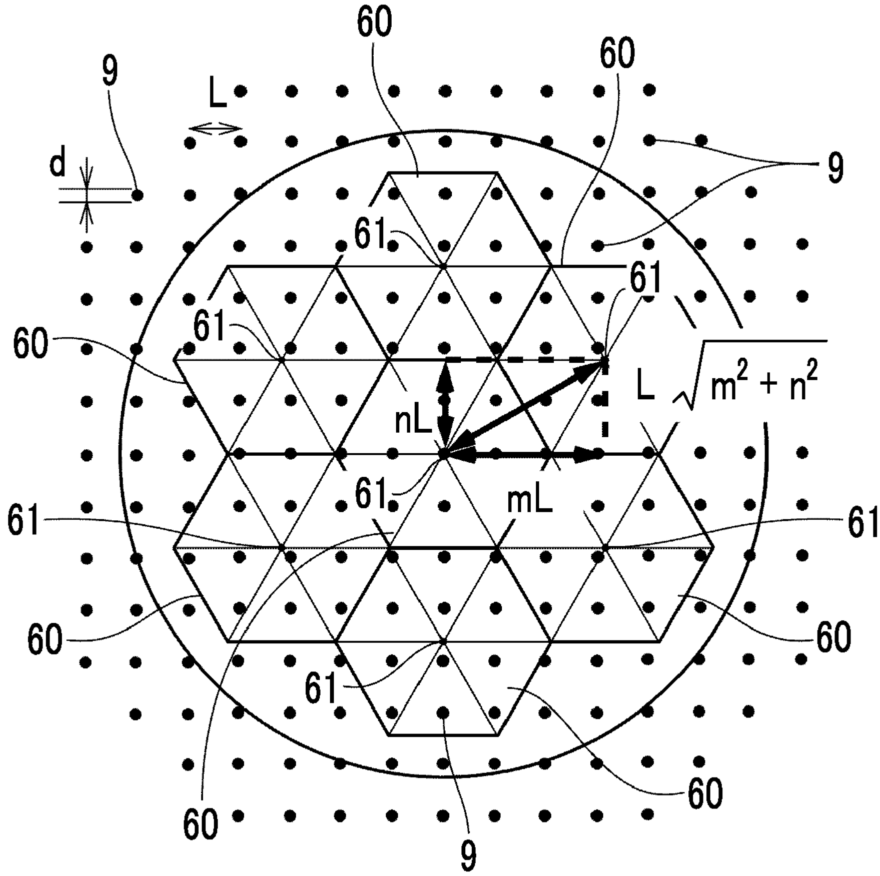
60
60
9
L
d
9
61
61
61
60
nL
$L\sqrt{m^2 + n^2}$
61
mL
61
61
60
60
61
60
60
9

FIG. 8

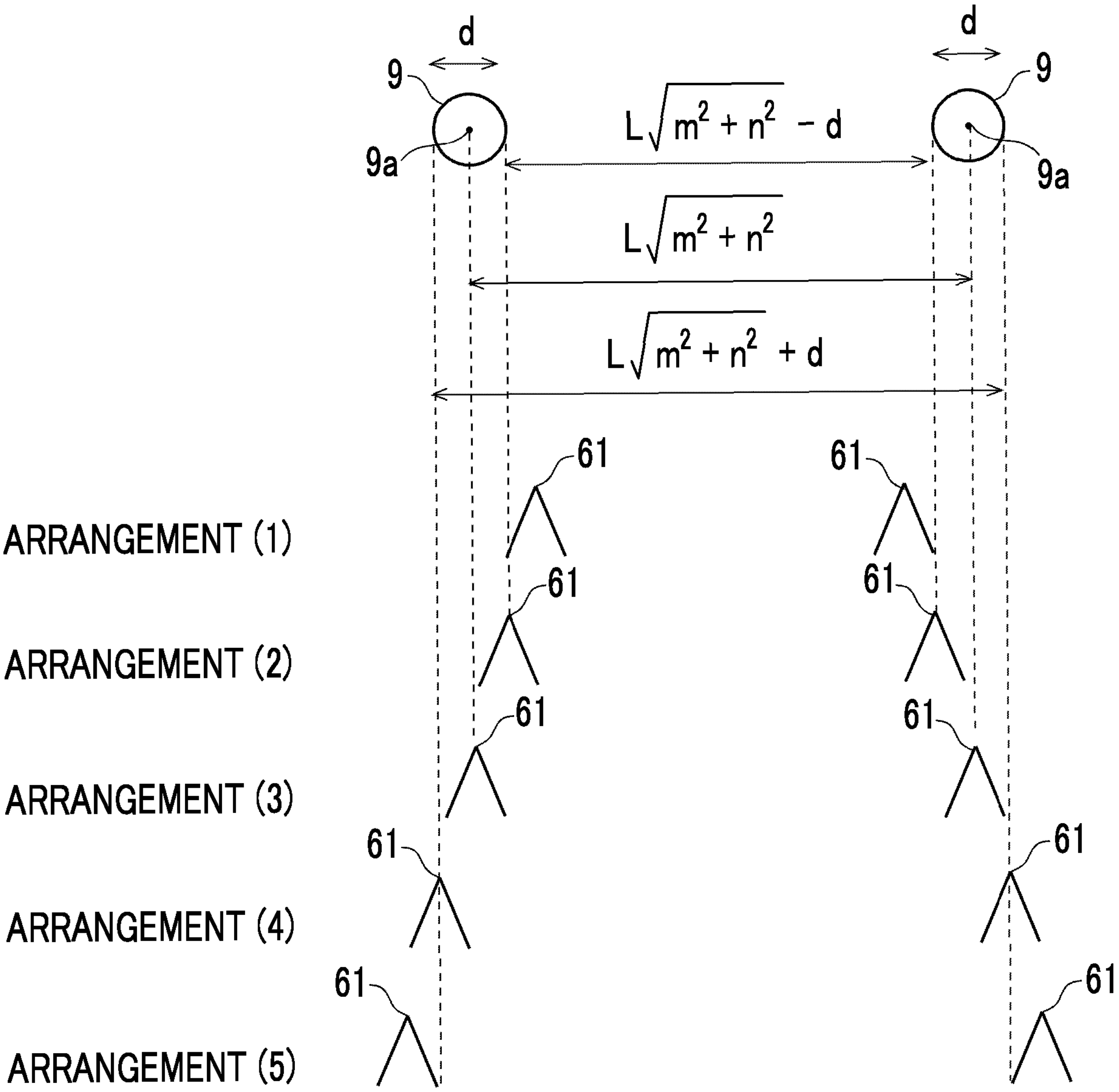
d
9
9a
$L\sqrt{m^2 + n^2} - d$
$L\sqrt{m^2 + n^2}$
$L\sqrt{m^2 + n^2} + d$
61
ARRANGEMENT (1)
ARRANGEMENT (2)
ARRANGEMENT (3)
ARRANGEMENT (4)
ARRANGEMENT (5)

FIG. 9

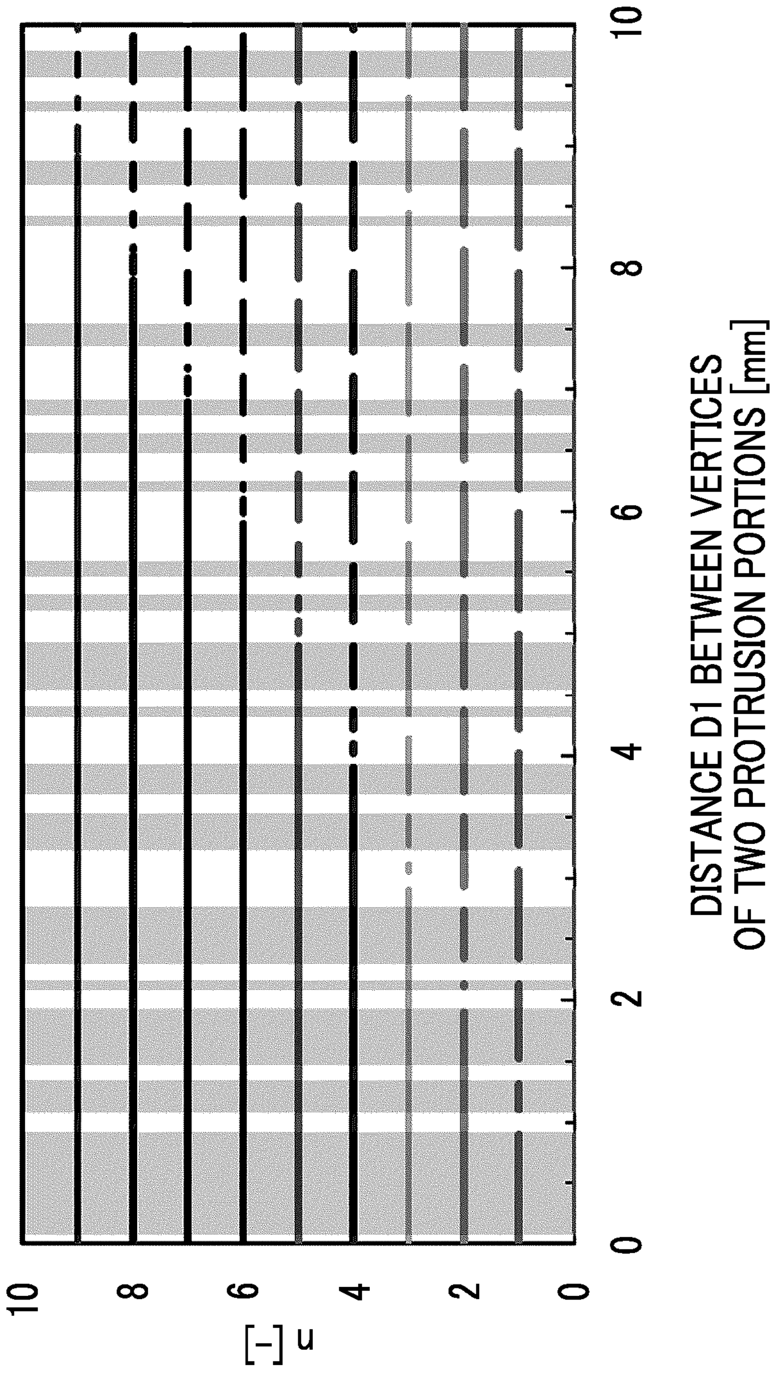
10
8
6
4
2
0
n [-]
0
2
4
6
8
10
DISTANCE D1 BETWEEN VERTICES
OF TWO PROTRUSION PORTIONS [mm]

FIG. 10A

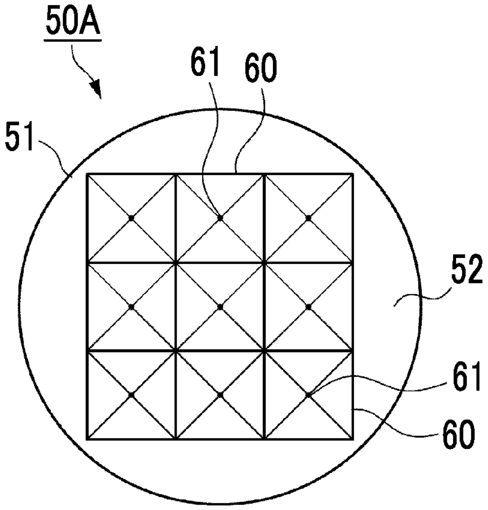


FIG. 10B

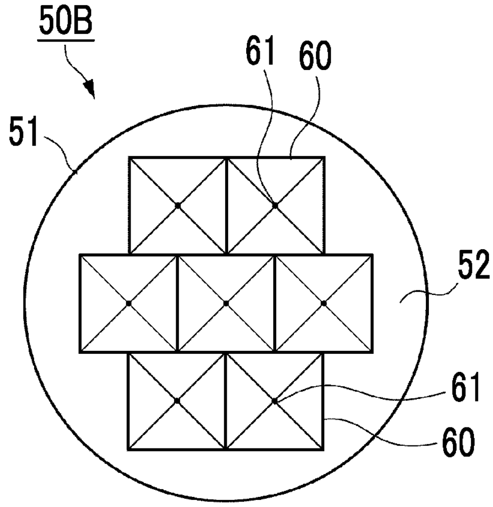


FIG. 10C

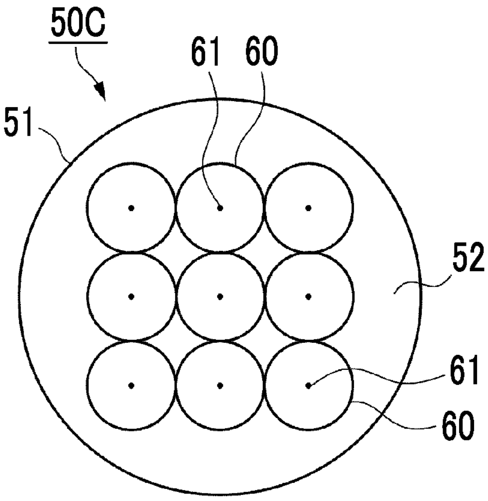


FIG. 10D

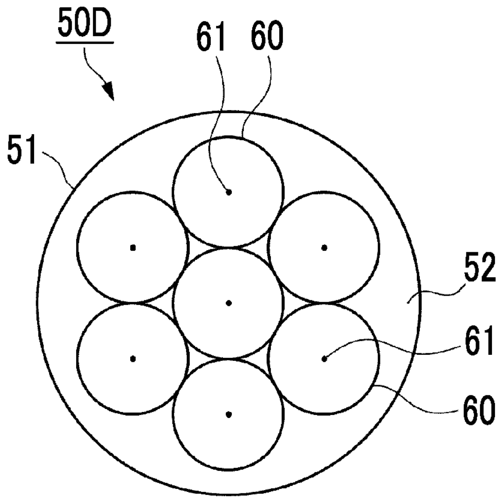


FIG. 10E

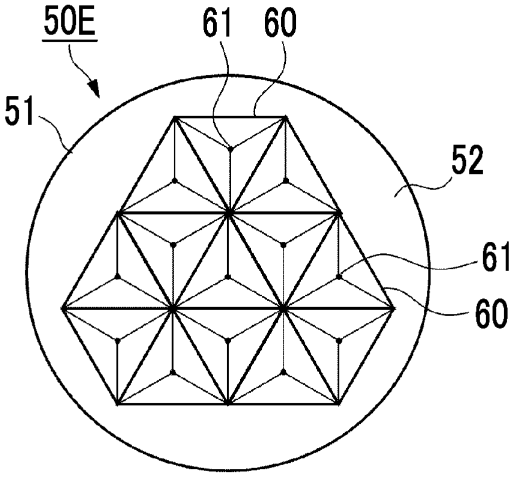

## INTERNATIONAL SEARCH REPORT

International application No.
**PCT/JP2022/034608**

**A. CLASSIFICATION OF SUBJECT MATTER**

***A61B 5/291***(2021.01)i; ***A61B 5/256***(2021.01)i
FI: A61B5/291; A61B5/256 110

According to International Patent Classification (IPC) or to both national classification and IPC

**B. FIELDS SEARCHED**

Minimum documentation searched (classification system followed by classification symbols)

A61B5/291; A61B5/256

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 4967038 A (SAM TECHOLOGY INC.) 30 October 1990 (1990-10-30) column 4, line 20 to column 9, line 27 | 1-10 |
| Y | JP 2015-027989 A (MANDOM CORP.) 12 February 2015 (2015-02-12) paragraphs [0009]-[0108] | 1-10 |
| Y | WO 2008/143131 A1 (LION CORP.) 27 November 2008 (2008-11-27) paragraphs [0016]-[0091] | 1-10 |
| Y | JP 2016-526972 A (NORTHEASTERN UNIV.) 08 September 2016 (2016-09-08) paragraphs [0020]-[0084] | 3 |
| Y | JP 3209880 U (HIRAK CORP.) 13 April 2017 (2017-04-13) paragraphs [0015]-[0049] | 7 |
| Y | WO 2020/080395 A1 (NOK CORP.) 23 April 2020 (2020-04-23) paragraphs [0021]-[0083] | 7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 November 2022** | **29 November 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/034608**

| Patent document cited in search report | Publication date (day/month/year) | Patent family member(s) | Publication date (day/month/year) |
|---|---|---|---|
| US 4967038 A | 30 October 1990 | WO 1992/002176 A1<br>pages 9-17<br>JP 63-226340 A | |
| JP 2015-027989 A | 12 February 2015 | (Family: none) | |
| WO 2008/143131 A1 | 27 November 2008 | CN 101677679 A<br>page 6, line 1 to page 22, line 5<br>KR 10-2010-0023798 A | |
| JP 2016-526972 A | 08 September 2016 | US 2016/0120432 A1<br>paragraphs [0043]-[0106]<br>WO 2014/205356 A2 | |
| JP 3209880 U | 13 April 2017 | (Family: none) | |
| WO 2020/080395 A1 | 23 April 2020 | US 2020/0268267 A1<br>paragraphs [0033]-[0096]<br>CN 111356403 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5842198 B
- JP 2021029118 A
- JP 2016136629 A
- JP 2021 A
- JP 155434 A